# EUROPEAN PATENT SPECIFICATION

(11) **EP 2 164 971 B1**
(45) Date of publication and mention of the grant of the patent: **09.12.2015**
(21) Application number: 08766339.9
(22) Date of filing: 16.06.2008
(51) Int. Cl.: C12N 15/11, C12N 15/85, C12N 9/64

(54) **METHOD FOR MANUFACTURING ACTIVE RECOMBINANT BLOOD COAGULATION FACTOR IX**
VERFAHREN ZUR HERSTELLUNG VON AKTIVEM REKOMBINANTEM BLUTGERINNUNGSFAKTOR IX
PROCÉDÉ DE FABRICATION DE FACTEUR IX DE LA COAGULATION SANGUINE RECOMBINANT ACTIF

(30) Priority: 15.06.2007 US 944186 P
(43) Date of publication of application: 24.03.2010
(73) Proprietor: Mogam Biotechnology Research Institute, Gyunggi-do 449-910 (KR)
(72) Inventor: KIM, Jung-Seob, Yongin-si Gyeonggi-do 446-799 (KR); LIM, Inhwan, Yongin-si Gyeonggi-do 446-799 (KR); CHOI, Murim, Yongin-si Gyeonggi-do 446-799 (KR); LEE, Gue-Hwa, Yongin-si Gyeonggi-do 446-799 (KR); YOON, Yeup, Yongin-si Gyeonggi-do 446-799 (KR)
(74) Representative: Grünecker Patent- und Rechtsanwälte PartG mbB
(86) International application number: PCT/KR2008/003377
(87) International publication number: WO 2008/153366

(56) References cited:
- WO-A2-00/03000
- WO-A2-2007/003582
- US-A- 5 935 935
- US-A- 6 046 380
- WAJIH N ET AL: "Increased production of functional recombinant human clotting factor IX by baby hamster kidney cells engineered to overexpress VKORC1, the vitamin K 2,3-epoxide-reducing enzyme of the vitamin K cycle" JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M505373200, vol. 280, no. 36, 19 July 2005 (2005-07-19), pages 31603-31607, XP002375182 ISSN: 0021-9258
- SUNG Y H ET AL: "Yeast hydrolysate as a low-cost additive to serum-free medium for the production of human thrombopoietin in suspension cultures of Chinese hamster ovary cells" APPLIED MICROBIOLOGY AND BIOTECHNOLOGY, SPRINGER VERLAG, BERLIN, DE LNKD- DOI:10.1007/S00253-003-1389-1, vol. 63, no. 5, 1 May 2004 (2004-05-01), pages 527-536, XP002555914 ISSN: 0175-7598 [retrieved on 2003-07-11]
- KURACHI S ET AL: 'Role of intron I in expression of the human factor IX gene' J. BIOL. CHEM. vol. 270, no. 10, 10 March 1995, USA, pages 5276 - 5281, XP002055415
- KURACHI S ET AL: 'Genetic Mechanisms of Age Regulation of Human Blood Coagulation Factor IX' SCIENCE vol. 285, no. 5428, 30 July 1999, USA, pages 739 - 743, XP002218779
- MIAO CH ET AL: 'Inclusion of the hepatic locus control regime, an intron, and untranslated region increases and stabilizes hepatic factor IX gene expression in vivo but not in vitro' MOL. THER. vol. 1, no. 6, June 2000, USA, pages 522 - 532, XP001063991
- EHRHARDT A ET AL: 'A new adenoviral helper-dependent vector results in long-term therapeutic levels of human coagulation factor IX at low doses in vivo' BLOOD vol. 99, no. 11, 01 June 2002, USA, pages 3923 - 3930, XP008129614
- DATABASE GENBANK [Online] 14 February 2003 XP008117987 Retrieved from NCBI Database accession no. (NG_000007)

<110> Mogam Biotechnology Research Institute
<120> METHOD FOR MANUFACTURING ACTIVE RECOMBINANT BLOOD COAGULATION FACTOR IX
<130> EP68507HV163pau
<140> 08 766 339.9
   <141> 2008-06-16
<150> PCT/KR2007/003377
   <151> 2008-06-16
<150> US 60/944,186
   <151> 2007-06-15
<160> 11
<170> KopatentIn 1.71
<210> 1
   <211> 2969
   <212> DNA
   <213> Artificial Sequence
<220>
   <223> DNA Sequence for Human beta-globin MAR Element
<400> 1
   cctcctgagt agctggggac tacaggtgta catcacatgc ctggctaatt tttttttttt 60
   taagtagaga cgaggtcttg ctatgttgtc caggataata tcaaactctt gagctcaagc 120
   agtcctccca cttctacctc tccagtgctg gaattacaga catgagccac cactcctggc 180
   ttgcagacta tttaaatgac taattcctga cactacttga gggatactag acagtagaca 240
   acacatcttt aatataccaa atgggtgact gtagggttga gagggagatt agaattcaat 300
   gttttatgac caaaaaggct taaatcaggc acaagcttag gtcttttcaa ctgtgaggac 360
   cggactgaaa gtgtgcagtt caaggccctg tagttgctgt ttaactgttc ccaggtggaa 420
   gtctcttcaa agaaccactg gtgcaaaaag ggaactacct ggggataaat atttcctcca 480
   gaaaggggga aagtgcaagc tcccctacca aaagcaccag gcaagtcctt gtctattttc 540
   cctgaagttc tcaaagaaat gagacccttg tttaccttta agattagaga aggcttgaaa 600
   agtttgagct gtgcctttgg aggccaacaa acttttctcc tttgttgacc aagttcagct 660
   ctcctgtata cttccaaggt ctgttgcatc aagagtgagt attgaaggtc ttagaagctg 720
   ggatctcaga tgtagggaaa agaggagatt tcctgttcac tcactgttaa gatatggctg 780
   aaattttttg atctagtcat ctacaaagca tgagttgtgg gtcagaaatt gtttttcaca 840
   tcttttgact tcctttgaca tcagaatata acctaggaat tgattactta agtgaaggca 900
   aggtactttg gtctggacag gaacattttg aacaaggtag ggagacagct atgaaggcaa 960
   gcatttattc tatctatcat ctatctgtct atctatctat tctttcatcc acttatttat 1020
   acatttaaac aaaaagtata gagcgtagta taatttgtaa gtgctcaggg ctgtgtgtgt 1080
   atggattgtt tgaaatgaaa ctaaagtggg agtataattc tactgccccc ttaaccctgt 1140
   ggtccctaca ctaccctgca agactcttag ctgcttagct taattgtgag gctgatttgg 1200
   ggcatagcac cccatcctct ctgtctttca acatcctcat aataacttga gaataatttt 1260
   ataaaatatc acaatagggt catgttcagt agggtgatat ataaaattag acaagccata 1320
   gtttgagttt acccttttga ataaatatat gacaaaaggc aatttaatta tctttatgag 1380
   tttggaggta tccagtatga aatttagata atacctgcct tctagtgttg aaattagaac 1440
   ttaatgatat aatgcatcaa tgaacttatt atagttccta gcacaaagta agaatccttt 1500
   caatgtgtgt gtgtgtgtat gtatttatct gttattaata ggaatcttat gggcattatc 1560
   tcacttaatc cttattaata actatgaagc aggtatttat ttgagttttc caagtgagtt 1620
   aagtatagct tgtaatactt aaggaaatat ccacaagtta catagctagt atataactga 1680
   gaaataattt tatttatatt ataaaacatt ctaacaatac agatgtatat aaactaaaaa 1740
   actgaaaggg ctcatgcaac cctaccttct caatatcact tcttcactta gaaaaaacca 1800
   gccttagctg tctgctatga atcctttcaa aatatacttc tgagaaatga gagagagaaa 1860
   tggggagggt agaaggaagg aagatagggt aagagacagg gaaggaggtg tggggaaaga 1920
   aattaaatta ttcttttctc tgtctcttga aagagctctt tccattacat tgaatcaaag 1980
   gtaatgttgc catttctgga ctcttgaaat aaagaaagac cgatgtatga aatgattttg 2040
   aaagtctatg gcattttcaa aatgcaaggt gatgtcttac taactagcct ttgctttatt 2100
   attagaaatg gggaagtgag tatagacatt ttatcaggag atatattagg aaaaagggaa 2160
   actggagaaa ctgggaggag tatccagatg tcctgtccct gtaaggtggg ggcacccacc 2220
   ttcaatcaaa agggctcctt aacaacttcc ttgcttgggg ctccaccatc ttggaccatt 2280
   agctccacag gtatcttctt ccctctagtg gtcataacag cagcttcagc tacctctcta 2340
   aagagtcctg ccagatatag gtcaggaaat ataatccact aataaaaaga gaaacatttt 2400
   gactgtagtt gtttgttttt tgtcattgtg actatcaata acattcccac tctttcatca 2460
   gtaatcactc aggttattct gtgaccaaca gactgtggga aaaatcagag aaggaggcat 2520
   cctcatgctt actagcctaa actgaaattg ctatagcaga gtgaaccaga aggtttacag 2580
   atattttcca caaagagtaa aaggattgaa gccttctcca gatcaatgca taggaaataa 2640
   taatggacca taaaacccat attatgacga acaacattag gataagtcca tatcaatttt 2700
   taatccagtc ataagcacag actacgtgaa gcacgtccaa gtgaaggcag gagaaatgag 2760
   aggagcaaga aagaggagcc atttgatcaa gaatagcaga aaaaggaaag gcaagtcata 2820
   ttaacaaatg attgtcatgc caacagtaca gataactctg ctaataaagg tagaggcata 2880
   atacaggtag tagcagatat ctacatagta gttaaaggac atggccatca gtacagaaga 2940
   ttccataaag gagaacctaa agaggaaaa 2969
<210> 2
   <211> 316
   <212> DNA
   <213> Artificial Sequence
<220>
   <223> DNA Sequence for Intron 1 Fragment of Blood Coagulation Factor IX
<400> 2
   gtttgtttgt cgaccctttt ttaaaataca ttgagtatgc ttgcctttta gatatagaaa 60
   tatctgatgc tgtcttcttc actaaatttt gattacatga tttgacagca atattgaaga 120
   gtctaacagc cagcacgcag gttggtaacg cgtgtactgt gggaacatca cagattttgg 180
   ctccatgccc taaagagaaa ttggctttca gattatttgg attaaaaaca aagactttct 240
   taagagatgt aaaattttca tgatgttttc ttttttgcta aaactaaaga attaactagt 300
   ttcttttaca tttcag 316
<210> 3
   <211> 34
   <212> DNA
   <213> Artificial Sequence
<220>
   <223> Forward primer for FIX
<400> 3
   ctagctagcc accatgcagc gcgtgaacat gatc 34
<210> 4
   <211> 33
   <212> DNA
   <213> Artificial Sequence
<220>
   <223> Reverse primer for FIX
<400> 4
   gaagatcttc attaagtgag ctttgttttt tcc 33
<210> 5
   <211> 8053
   <212> DNA
   <213> Artificial sequence
<220>
   <223> DNA Sequence for pMSG-FIXI Vector Encoding Blood coagulation Factor IX
<400> 5
   gcgcagcacc atggcctgaa ataacctctg aaagaggaac ttggttaggt accttctgag 60
   gcggaaagaa ccagctgtgg aatgtgtgtc agttagggtg tggaaagtcc ccaggctccc 120
   cagcaggcag aagtatgcaa agcatgcatc tcaattagtc agcaaccagg tgtggaaagt 180
   ccccaggctc cccagcaggc agaagtatgc aaagcatgca tctcaattag tcagcaacca 240
   tagtcccgcc cctaactccg cccatcccgc ccctaactcc gcccagttcc gcccattctc 300
   cgccccatgg ctgactaatt ttttttattt atgcagaggc cgaggccgcc tcggcctctg 360
   agctattcca gaagtagtga ggaggctttt ttggaggcct aggcttttgc aaaaagcttg 420
   ctagccacca tgcagcgcgt gaacatgatc atggcagaat caccaggcct catcaccatc 480
   tgccttttag gatatctact cagtgctgaa tgtacaggtt tgtttgtcga ccctttttta 540
   aaatacattg agtatgcttg ccttttagat atagaaatat ctgatgctgt cttcttcact 600
   aaattttgat tacatgattt gacagcaata ttgaagagtc taacagccag cacgcaggtt 660
   ggtaacgcgt gtactgtggg aacatcacag attttggctc catgccctaa agagaaattg 720
   gctttcagat tatttggatt aaaaacaaag actttcttaa gagatgtaaa attttcatga 780
   tgttttcttt tttgctaaaa ctaaagaatt aactagtttc ttttacattt cagtttttct 840
   tgatcatgaa aacgccaaca aaattctgaa tcggccaaag aggtataatt caggtaaatt 900
   ggaagagttt gttcaaggga accttgagag agaatgtatg gaagaaaagt gtagttttga 960
   agaagcacga gaagtttttg aaaacactga aagaacaact gaattttgga agcagtatgt 1020
   tgatggagat cagtgtgagt ccaatccatg tttaaatggc ggcagttgca aggatgacat 1080
   taattcctat gaatgttggt gtccctttgg atttgaagga aagaactgtg aattagatgt 1140
   aacatgtaac attaagaatg gcagatgcga gcagttttgt aaaaatagtg ctgataacaa 1200
   ggtggtttgc tcctgtactg agggatatcg acttgcagaa aaccagaagt cctgtgaacc 1260
   agcagtgcca tttccatgtg gaagagtttc tgtttcacaa acttctaagc tcacccgtgc 1320
   tgaggctgtt tttcctgatg tggactatgt aaattctact gaagctgaaa ccattttgga 1380
   taacatcact caaagcaccc aatcatttaa tgacttcact cgggttgttg gtggagaaga 1440
   tgccaaacca ggtcaattcc cttggcaggt tgttttgaat ggtaaagttg atgcattctg 1500
   tggaggctct atcgttaatg aaaaatggat tgtaactgct gcccactgtg ttgaaactgg 1560
   tgttaaaatt acagttgtcg caggtgaaca taatattgag gagacagaac atacagagca 1620
   aaagcgaaat gtgattcgaa ttattcctca ccacaactac aatgcagcta ttaataagta 1680
   caaccatgac attgcccttc tggaactgga cgaaccctta gtgctaaaca gctacgttac 1740
   acctatttgc attgctgaca aggaatacac gaacatcttc ctcaaatttg gatctggcta 1800
   tgtaagtggc tggggaagag tcttccacaa agggagatca gctttagttc ttcagtacct 1860
   tagagttcca cttgttgacc gagccacatg tcttcgatct acaaagttca ccatctataa 1920
   caacatgttc tgtgctggct tccatgaagg aggtagagat tcatgtcaag gagatagtgg 1980
   gggaccccat gttactgaag tggaagggac cagtttctta actggaatta ttagctgggg 2040
   tgaagagtgt gcaatgaaag gcaaatatgg aatatatacc aaggtatccc ggtatgtcaa 2100
   ctggattaag gaaaaaacaa agctcactta atgaagatct gttaactcga gaacttgttt 2160
   attgcagctt ataatggtta caaataaagc aatagcatca caaatttcac aaataaagca 2220
   tttttttcac tgcattctag ttgtggtttg tccaaactca tcaatgtatc ttatcatgtc 2280
   tggatccagg ataatatatg gtagggttca tagccagagt aacctttttt tttaattttt 2340
   attttatttt attttgagct gcaggcatgc aagctggcac tggccgtcgt tttacaacgt 2400
   cgtgactggg aaaaccctgg cgttacccaa cttaatcgcc ttgcagcaca tccccctttc 2460
   gccagctggc gtaatagcga agaggcccgc accgatcgcc cttcccaaca gttgcgcagc 2520
   ctgaatggcg aatggcgcct gatgcggtat tttctcctta cgcatctgtg cggtatttca 2580
   caccgcatat ggtgcactct cagtacaatc tgctctgatg ccgcatagtt aagccagccc 2640
   cgacacccgc caacacccgc tgacgcgccc tgacgggctt gtctgctccc ggcatccgct 2700
   tacagacaag ctgtgaccgt ctccgggagc tgcatgtgtc agaggttttc accgtcatca 2760
   ccgaaacgcg cgagacgaaa gggcctcgtg atacgcctat ttttataggt taatgtcatg 2820
   ataataatgg tttcttagac gtcaggtggc acttttcggg gaaatgtgcg cggaacccct 2880
   atttgtttat ttttctaaat acattcaaat atgtatccgc tcatgagaca ataaccctga 2940
   taaatgcttc aataatattg aaaaaggaag agtatgagta ttcaacattt ccgtgtcgcc 3000
   cttattccct tttttgcggc attttgcctt cctgtttttg ctcacccaga aacgctggtg 3060
   aaagtaaaag atgctgaaga tcagttgggt gcacgagtgg gttacatcga actggatctc 3120
   aacagcggta agatccttga gagttttcgc cccgaagaac gttttccaat gatgagcact 3180
   tttaaagttc tgctatgtgg cgcggtatta tcccgtattg acgccgggca agagcaactc 3240
   ggtcgccgca tacactattc tcagaatgac ttggttgagt actcaccagt cacagaaaag 3300
   catcttacgg atggcatgac agtaagagaa ttatgcagtg ctgccataac catgagtgat 3360
   aacactgcgg ccaacttact tctgacaacg atcggaggac cgaaggagct aaccgctttt 3420
   ttgcacaaca tgggggatca tgtaactcgc cttgatcgtt gggaaccgga gctgaatgaa 3480
   gccataccaa acgacgagcg tgacaccacg atgcctgtag caatggcaac aacgttgcgc 3540
   aaactattaa ctggcgaact acttactcta gcttcccggc aacaattaat agactggatg 3600
   gaggcggata aagttgcagg accacttctg cgctcggccc ttccggctgg ctggtttatt 3660
   gctgataaat ctggagccgg tgagcgtggg tctcgcggta tcattgcagc actggggcca 3720
   gatggtaagc cctcccgtat cgtagttatc tacacgacgg ggagtcaggc aactatggat 3780
   gaacgaaata gacagatcgc tgagataggt gcctcactga ttaagcattg gtaactgtca 3840
   gaccaagttt actcatatat actttagatt gatttaaaac ttcattttta atttaaaagg 3900
   atctaggtga agatcctttt tgataatctc atgaccaaaa tcccttaacg tgagttttcg 3960
   ttccactgag cgtcagaccc cgtagaaaag atcaaaggat cttcttgaga tccttttttt 4020
   ctgcgcgtaa tctgctgctt gcaaacaaaa aaaccaccgc taccagcggt ggtttgtttg 4080
   ccggatcaag agctaccaac tctttttccg aaggtaactg gcttcagcag agcgcagata 4140
   ccaaatactg ttcttctagt gtagccgtag ttaggccacc acttcaagaa ctctgtagca 4200
   ccgcctacat acctcgctct gctaatcctg ttaccagtgg ctgctgccag tggcgataag 4260
   tcgtgtctta ccgggttgga ctcaagacga tagttaccgg ataaggcgca gcggtcgggc 4320
   tgaacggggg gttcgtgcac acagcccagc ttggagcgaa cgacctacac cgaactgaga 4380
   tacctacagc gtgagctatg agaaagcgcc acgcttcccg aagggagaaa ggcggacagg 4440
   tatccggtaa gcggcagggt cggaacagga gagcgcacga gggagcttcc agggggaaac 4500
   gcctggtatc tttatagtcc tgtcgggttt cgccacctct gacttgagcg tcgatttttg 4560
   tgatgctcgt caggggggcg gagcctatgg aaaaacgcca gcaacgcggc ctttttacgg 4620
   ttcctggcct tttgctggcc ttttgctcac atgttctttc ctgcgttatc ccctgattct 4680
   gtggataacc gtattaccgc ctttgagtga gctgataccg ctcgccgcag ccgaacgacc 4740
   gagcgcagcg agtcagtgag cgaggaagcg gaagagcgcc caatacgcaa accgcctctc 4800
   cccgcgcgtt ggccgattca ttaatgcagc tggcacgaca ggtttcccga ctggaaagcg 4860
   ggcagtgagc gcaacgcaat taatgtgagt tagctcactc attaggcacc ccaggcttta 4920
   cactttatgc ttccggctcg tatgttgtgt ggaattgtga gcggataaca atttcacaca 4980
   ggaaacagct atgaccatga ttacgccaag cgcgcaatta accctcacta aagggaacaa 5040
   aagctggagc tccaccgcgg cccgggcctc ctgagtagct ggggactaca ggtgtacatc 5100
   acatgcctgg ctaatttttt tttttttaag tagagacgag gtcttgctat gttgtccagg 5160
   ataatatcaa actcttgagc tcaagcagtc ctcccacttc tacctctcca gtgctggaat 5220
   tacagacatg agccaccact cctggcttgc agactattta aatgactaat tcctgacact 5280
   acttgaggga tactagacag tagacaacac atctttaata taccaaatgg gtgactgtag 5340
   ggttgagagg gagattagaa ttcaatgttt tatgaccaaa aaggcttaaa tcaggcacaa 5400
   gcttaggtct tttcaactgt gaggaccgga ctgaaagtgt gcagttcaag gccctgtagt 5460
   tgctgtttaa ctgttcccag gtggaagtct cttcaaagaa ccactggtgc aaaaagggaa 5520
   ctacctgggg ataaatattt cctccagaaa gggggaaagt gcaagctccc ctaccaaaag 5580
   caccaggcaa gtccttgtct attttccctg aagttctcaa agaaatgaga cccttgttta 5640
   cctttaagat tagagaaggc ttgaaaagtt tgagctgtgc ctttggaggc caacaaactt 5700
   ttctcctttg ttgaccaagt tcagctctcc tgtatacttc caaggtctgt tgcatcaaga 5760
   gtgagtattg aaggtcttag aagctgggat ctcagatgta gggaaaagag gagatttcct 5820
   gttcactcac tgttaagata tggctgaaat tttttgatct agtcatctac aaagcatgag 5880
   ttgtgggtca gaaattgttt ttcacatctt ttgacttcct ttgacatcag aatataacct 5940
   aggaattgat tacttaagtg aaggcaaggt actttggtct ggacaggaac attttgaaca 6000
   aggtagggag acagctatga aggcaagcat ttattctatc tatcatctat ctgtctatct 6060
   atctattctt tcatccactt atttatacat ttaaacaaaa agtatagagc gtagtataat 6120
   ttgtaagtgc tcagggctgt gtgtgtatgg attgtttgaa atgaaactaa agtgggagta 6180
   taattctact gcccccttaa ccctgtggtc cctacactac cctgcaagac tcttagctgc 6240
   ttagcttaat tgtgaggctg atttggggca tagcacccca tcctctctgt ctttcaacat 6300
   cctcataata acttgagaat aattttataa aatatcacaa tagggtcatg ttcagtaggg 6360
   tgatatataa aattagacaa gccatagttt gagtttaccc ttttgaataa atatatgaca 6420
   aaaggcaatt taattatctt tatgagtttg gaggtatcca gtatgaaatt tagataatac 6480
   ctgccttcta gtgttgaaat tagaacttaa tgatataatg catcaatgaa cttattatag 6540
   ttcctagcac aaagtaagaa tcctttcaat gtgtgtgtgt gtgtatgtat ttatctgtta 6600
   ttaataggaa tcttatgggc attatctcac ttaatcctta ttaataacta tgaagcaggt 6660
   atttatttga gttttccaag tgagttaagt atagcttgta atacttaagg aaatatccac 6720
   aagttacata gctagtatat aactgagaaa taattttatt tatattataa aacattctaa 6780
   caatacagat gtatataaac taaaaaactg aaagggctca tgcaacccta ccttctcaat 6840
   atcacttctt cacttagaaa aaaccagcct tagctgtctg ctatgaatcc tttcaaaata 6900
   tacttctgag aaatgagaga gagaaatggg gagggtagaa ggaaggaaga tagggtaaga 6960
   gacagggaag gaggtgtggg gaaagaaatt aaattattct tttctctgtc tcttgaaaga 7020
   gctctttcca ttacattgaa tcaaaggtaa tgttgccatt tctggactct tgaaataaag 7080
   aaagaccgat gtatgaaatg attttgaaag tctatggcat tttcaaaatg caaggtgatg 7140
   tcttactaac tagcctttgc tttattatta gaaatgggga agtgagtata gacattttat 7200
   caggagatat attaggaaaa agggaaactg gagaaactgg gaggagtatc cagatgtcct 7260
   gtccctgtaa ggtgggggca cccaccttca atcaaaaggg ctccttaaca acttccttgc 7320
   ttggggctcc accatcttgg accattagct ccacaggtat cttcttccct ctagtggtca 7380
   taacagcagc ttcagctacc tctctaaaga gtcctgccag atataggtca ggaaatataa 7440
   tccactaata aaaagagaaa cattttgact gtagttgttt gttttttgtc attgtgacta 7500
   tcaataacat tcccactctt tcatcagtaa tcactcaggt tattctgtga ccaacagact 7560
   gtgggaaaaa tcagagaagg aggcatcctc atgcttacta gcctaaactg aaattgctat 7620
   agcagagtga accagaaggt ttacagatat tttccacaaa gagtaaaagg attgaagcct 7680
   tctccagatc aatgcatagg aaataataat ggaccataaa acccatatta tgacgaacaa 7740
   cattaggata agtccatatc aatttttaat ccagtcataa gcacagacta cgtgaagcac 7800
   gtccaagtga aggcaggaga aatgagagga gcaagaaaga ggagccattt gatcaagaat 7860
   agcagaaaaa ggaaaggcaa gtcatattaa caaatgattg tcatgccaac agtacagata 7920
   actctgctaa taaaggtaga ggcataatac aggtagtagc agatatctac atagtagtta 7980
   aaggacatgg ccatcagtac agaagattcc ataaaggaga acctaaagag gaaaaatcga 8040
   taagcttgat ccc 8053
<210> 6
   <211> 29
   <212> DNA
   <213> Artificial Sequence
<220>
   <223> Forward primer for 5' fragment of FIX intron I
<400> 6
   gcgtcgaccc ttttttaaaa tacattgag 29
<210> 7
   <211> 28
   <212> DNA
   <213> Artificial sequence
<220>
   <223> Reverse primer for 5' fragment of FIX intron I
<400> 7
   cgacgcgtta ccaacctgcg tgctggct 28
<210> 8
   <211> 28
   <212> DNA
   <213> Artificial Sequence
<220>
   <223> Forward primer for 3' fragment of FIX intron I
<400> 8
   cgacgcgtgt actgtgggaa catcacag 28
<210> 9
   <211> 28
   <212> DNA
   <213> Artificial sequence
<220>
   <223> Reverse primer for 5' fragment of FIX intron I
<400> 9
   gactagttaa ttctttagtt ttagcaaa 28
<210> 10
   <211> 32
   <212> DNA
   <213> Artificial Sequence
<220>
   <223> Reverse primer for 5' fragment of FIX in pMSG-FIXI
<400> 10
   gcgtcgacaa acaaacctgt acattcagca ct 32
<210> 11
   <211> 40
   <212> DNA
   <213> Artificial Sequence
<220>
   <223> Forward primer for 3' fragment of FIX in pMSG-FIXI
<400> 11
   gactagtttc ttttacattt cagtttttct tgatcatgaa 40

## Description

### Field of the Invention

The invention relates to a method for manufacturing recombinant human blood coagulation factor IX (FIX).

### Background of the Invention

Hemophilia is a genetic disease caused by a disorder in the coagulation system associated with the X chromosome, and it is divided into 2 groups: hemophilia A caused by the lack of blood coagulation factor VIII, and hemophilia B caused by the lack of blood coagulation factor IX (FIX). About 80% of the hemophilia patients suffer from hemophilia A, and 20%, from hemophilia B.

A hemophilia patient must be continuously administered with the lacking blood coagulation factor to prevent or treat the bleeding. Therefore, an appropriate blood coagulation factor having proven stability has to be available, and recombinant FIX produced using recombinant genetic engineering technique and serum-free medium has recently been provided.

Generally, a recombinant FIX is obtained by culturing a CHO (Chinese hamster ovary) cell line in an optimized serum-free medium. However, because the levels of PACE (paired basic amino acid converting enzyme) and γ-carboxylase are not sufficiently high to act on the entire overexpressed FIX recombinant protein, only a part of the produced FIX gains activity. Consequently, the supernatant of such a culture contains, in addition to the desired aFIX, many FIX types which do not have blood coagulation activity such as uncarboxylated FIX, uncarboxylated pro-FIX, and carboxylated pro-FIX but having an unremoved propeptide residue. In order to solve this problem, there has been developed a CHO cell line modified to produce the recombinant FIX having more activity by incorporating human γ-carboxylase or PACE protease hereinto.

Also, a part of the secreted FIX is modified to become activated FIX (FIXa) depending on the cell culture condition or culture time, which makes it very difficult to enhance the FIX productivity and also to purify the FIX product having inactive FIX types or FIXa. Therefore, there has been a need to develop a cell line and culture process, which can minimize the secretion of unwanted FIX types and maximize the productivity of aFIX.

Most protein medicines such as EPO (erythropoietin) and therapeutic antibodies are produced using an animal cell culture method, primarily because an animal cell can efficiently produce a human-like glycosylation protein, in contrast to a bacterium, yeast or insect cell which has no or limited glycosylation capacity. The animal cell line used industrially to produce a recombinant medicine is represented by a CHO cell and a BHK (Baby hamster kidney) cell, which express a high level of a desired recombinant protein, and undergoes a post-translational modification of the expressed protein similar to that of the human protein.

In such animal cell culture, a serum used to be employed, but to avoid infectious factors derived from an animal serum, a serum-free medium has been developed. WO 2007/003582 describes the production of recombinant hFIX in 293F cells using unmodified serum-free medium. However, the productivity of the recombinant protein using such a serum-free medium is low, and therefore, culture additives such as a hydrolysate of a non-animal origin and a recombinant protein are added to the serum-free medium. Specifically, a medium using a vegetable or yeast hydrolysate has been developed (*see* WO 96/15231, WO 98/15614, WO 01/23527 and WO 00/03000), but the protein productivity has been found to be low due to the lack of essential nutrients required for producing the recombinant protein. Therefore, there has been attempts to add proteins derived from an animal to the serum-free medium.

Despite the development of the various culture mediums, there has remained a need to develop an optimal cell culture medium and culture method to enhance the expression efficiency and productivity of a desired recombinant protein.

### Summary of the Invention

The present invention relates to the following items:
1. A method for manufacturing human blood coagulation factor IX (FIX) by culturing an animal cell transfected with a recombinant expression vector expressing human FIX in a serum-free medium for animal cell culture, wherein the serum-free medium contains a yeast hydrolysate at a concentration of 2.5 to 5 g/L, and a calcium ion at a concentration of 1.0 to 1.3 mM, and has an osmotic pressure ranging from 350 to 388 mOsm.
2. The method of item 1, wherein the recombinant expression vector comprises:
   a nucleotide sequence of human β-globin MAR element having the nucleotide sequence of SEQ ID NO: 1; and
   a nucleotide sequence of human FIX having a fragment of intron 1 in place of the inherent intron 1 of the nucleotide sequence encoding human FIX, wherein the fragment of intron 1 is composed of a 141 bp to 981 bp long nucleotide sequence having the 5'-end splicing donor of intron 1 fused with a 157 bp to 444 bp long nucleotide sequence having the 3'-end splicing acceptor of intron 1; and the fragment of intron 1 is a 316 bp long nucleotide sequence.
3. The method of item 2, wherein the fragment of intron 1 has the nucleotide sequence of SEQ ID NO: 2.
4. The method of item 2, wherein the recombinant expression vector has the nucleotide sequence of SEQ ID NO: 5.
5. The method of item 1, wherein the animal cell is CHO, BHK or COS7.
6. The method of item 1, wherein the animal cell is further transfected with at least one vector selected from the group consisting of expression vectors expressing furin, secretor type furin and carboxylase, respectively.
7. A serum-free medium for manufacturing human blood coagulation factor IX (FIX), which is a serum-free medium for animal cell culture having a yeast hydrolysate at a concentration of 2.5 to 5 g/L, an osmotic pressure ranging from 350 to 388 mOsm, and a calcium ion at a concentration of 1.0 to 1.3 mM.

### Brief Description of Drawings

The above and other objects and features of the present invention will become apparent from the following description of the invention taken in conjunction with the following accompanying drawings, which respectively show:
Fig. 1A: diagrams of FIX expression vectors, pMSG-FIX and pMSG-FIXI;
Fig. 1B: diagrams of expression vectors for furin, secretor type furin and γ-carboxylase cloned into vector pMSG, respectively;
Fig. 2: the enhancement effect of the FIX expression in an animal cell line transfected with a vector comprising a fragment of intron 1 of FIX;
Fig. 3: the amount of the FIX expression according to a recombinant FIX expression vector transfected into a cell line adapted to 1 µM MTX;
Figs. 4A and 4B: the cell growth of each of the FIX producing cell lines in a culture with a serum-free medium, as well as the activity and productivity of the FIX;
Fig. 4C: the cell growth and viability of F24 cell line (A), the productivity (B) and the activity (C) of the FIX, and immuno blotting for the FIX (D) according to the serum-free suspension culture using GC-P1 serum-free medium;
Fig. 5A: the cell growth of F24 cell line according to the culture time after adding yeast hydrolysate (Y), soy hydrolysate (S) or wheat hydrolysate (WG) to a serum-free GC-P1 medium (SFM);
   Fig. 5B: the relative productivity of the FIX after adding yeast hydrolysate (Y), soy hydrolysate (S) or wheat hydrolysate (WG) to the serum-free GC-P1 medium (SFM), as compared with the productivity using only GC-P1 medium;
Fig. 6A: the cell growth of F24 cell lines in the culture obtainded by adding yeast hydrolysate (Y) and soy hydrolysate (S) in a various mix ratio to the serum-free GC-P1 medium (SFM);
Fig. 6B: the relative productivity of the FIX in the culture obtainded by adding yeast hydrolysate (Y) and soy hydrolysate (S) in a various mix ratio to the serum-free GC-P1 medium (SFM), as compared with the productivity using only the GC-P1 medium;
Fig. 7A: the cell growth of F24 cell line in the serum-free culture having yeast hydrolysate added in a controlled amount at a controlled time; and
Fig. 7B: the activity of FIX obtained from F24 cell line in the serum-free culture having yeast hydrolysate added in a controlled amount at a controlled time.

### Detailed Description of the Invention

The present invention describes an expression vector which expresses a human recombinant FIX characterized in that it comprises a human β-globin MAR element and a fragment of intron 1 of human FIX.

The human β-globin MAR element (*see* Korea Patent No. 10-0408844; SEQ ID NO: 1) is a matrix attachment region element (MAR element) known as a nucleic acid factor which controls gene replication and expression in an animal cell. In the expression of a recombinant protein using an animal cell, the element is used to overcome the problem that the protein expression level varies depending on the gene insertion position. By using the vector comprising the element, the protein expression level may be maximized, and the time required for obtaining the final cell line can be reduced to 1-2 months because the expression gene of the recombinant protein is easily amplified. As a vector comprising the human β-globin MAR element, the known vector pMSG (Accession No.: KCCM 10202) may be used.

As described in the present invention, a fragment of human FIX intron 1 is used to increase the expression level (Kurachi, S. et al comprising. Journal of biological chemistry 270:5276-5281, 1995). The fragment is a gene fragment prepared by connecting a 141 bp to 981 bp long segment containing the splicing donor located at the 5'-end of the FIX intron 1 and a 157 bp to 444 bp long segment containing the splicing acceptor located at the 3'-end thereof. Preferably, it is a gene fragment prepared by connecting a 141 bp long segment containing the splicing donor at the 5'-end of the FIX intron 1 and a 157 bp long segment containing the splicing acceptor at the 3'-end thereof.

The intron 1 fragment is cloned into the inherent intron 1 site of FIX gene. The fragment of intron 1 preferably has 298 bp to 1425 bp long nucleotide sequence. More preferably, the fragment has a 316 bp long nucleotide sequence containing a 298 bp long intron 1 fragment and a fused region for cloning. Specifically, the intron 1 fragment may have the nucleotide sequence of SEQ ID NO: 2.

By using the FIX expression vector comprising the MAR element together with the intron 1 fragment, the FIX expression level may increase about 10 times relative to that achievable using an expression vector lacking the intron 1 fragment. Specifically, the specific productivity (a value representing the cell productivity as the productivity per cell in a unit time) of cell lines transfected with the FIX expression vector is very high, about 8 ~ 14 *µ*g/10⁶ cells/day.

The animal cell used in the method for animal cell culture of the present invention may be any of the CHO, BHK and COS7 cell lines known in the art. As CHO cell line, DG44 may be used, which is a cell line lacking dihydrofolate reductase (DHFR), which is involved in the nucleic acid synthesis. Therefore, the production of the recombinant human FIX as a target material may be amplified by: simultaneously introducing a plasmid having DHFR gene and a plasmid having a target protein gene into a CHO cell; culturing the cell line in a nucleic acid-free selection medium to select a recombinant cell line; and adding methotrexate (MTX), an analogue of folate used as a substrate of DHFR enzyme to the medium to induce the amplification of the introduced DHFR gene as well as that of the introduced target gene.

Further, the FIX expression vector may be transfected into a CHO cell and the like independently, or together with human furin, secretor-type furin (a region of lumen side of Golgi body of the furin enzyme except a transmembrane region of 3'-end and cytoplasmic tail region of the furin enzyme) or γ-carboxylase expression vector. The cell line highly expressing the recombinant FIX is isolated from the cell line transfected with the FIX expression vector, and is used in a serum-free culture.

As described in the present invention, the recombinant FIX expression cell line is subjected to suspension culture in GC-P1 serum-free medium of Green Cross Corp. and other known commercial serum-free media (e.g., serum-free medium of Hyclone and Sigma) to prepare a cell line adapted to suspension culture.

Specifically, FS09, F24 and GFSC-15 cell lines showed markedly high productivities among the recombinant FIX expression cell lines. The specific activity of the produced recombinant FIX was 20 *~* 40 IU/mg for FS09 and F24 cell lines, and 50 ~ 150 IU/mg for G 12 and GFSC-15 cell lines. As the specific activity of the FIX is at the level of 5 ug/ml in blood is represented commonly as 200 IU/mg, about 10 ~ 50% of the FIX secreted to a culture supernatant by culturing the cell line obtained is an active form of FIX. In this experiment, the GC-P1 serum-free medium for producing the FIX may be used as a serum-free suspension culture medium. Further, F24 and GFSC-15 cell lines, which show the high productivities and specific activities of the FIX in the GC-P1 serum-free medium, are selected as the final cell lines.

Further, the present invention describes a method for enhancing the productivity of the recombinant FIX by adding a soy hydrolysate, yeast hydrolysate or a combination thereof. As described herein, when the soy and yeast hydrolysates are added from the early stage of culture or at the exponential period of the cell growth, the hydrolysates enhance the cell growth or longevity at the late stage of culture, while they do not affect the cell growth at the early stage of the culture.

Further, when the soy hydrolysate or yeast hydrolysate is used alone or in combination, the FIX productivity increases about 1.8~2.0 times, as compared with the productivity observed in the GC-P1 serum-free medium. Preferably, the amount of the hydrolysate is 1~10 g/L, more preferably 2.5~5 g/L, based on the amount of the medium. When the yeast hydrolysate is used at a concentration of 1 ~ 10 g/L, preferably 2.5~5 g/L in culture, and when the concentration of early yeast hydrolysate addition adjust while controlling the addition time point of the hydrolysate, the productivity of the FIX may increase by about 70~100%.

For enhancing the cell growth and productivity, it is preferably to use the yeast hydrolysate alone. The time point for adding the hydrolysate may be from the early stage of the culture, and when the hydrolysate is added at the exponential period of the cell culture (3~4 days after culture), the FIX productivity may be maximized due to increased period of the cell growth and cell survival.

At the end of the serum-free culture, a part of the aFIX produced by the cell death and cell secretion is activated to generate undesirable activated FIX (FIXa). In order to inhibit the activation, the present invention describes means to inhibit the production of the FIXa by making the osmotic pressure of the culture medium high by using NaCl. The desirable range of the elevated osmotic pressure is 312~533 mOsm, and preferably 350~400 mOsm. The most preferable osmotic pressure for the maximum production of desired aFIX is about 388 mOsm.

The present invention describes also another means to maximize the production of aFIX in a culture medium during the serum-free culture by adding a yeast hydrolysate to GC-P1 basic medium in which the calcium ion (Ca²⁺) concentration related to the FIX activity is controlled by adding calcium chloride (CaCl₂). The total productivity of aFIX may be enhanced up to a of calcium chloride concentration of 2.6 mM, and the optimum concentration of the calcium ion to maximize the FIX production is about 1.0~1.3 mM under the condition that aFIX is not activated to FIXa.

Further, the present invention describes an optimal culture process to produce aFIX using a serum-free culture of an animal cell line producing FIX by applying the above mentioned means in combination.

Hereinafter, the present invention will be described more in detail with reference to examples, but the following Examples are intended to illustrate the present invention without limiting its scope.

### Test Example: Quantification of recombinant FIX and measurement of activity thereof

The expression level of the FIX expressed in a culture supernatant of the FIX producing cell line was measured by a sandwich ELISA method (Affinity Bio-logicals, Canada), and compared and quantitified by using a human plasma derived-FIX (Heamatologic Technologies Inc., USA) as a standard. The activity of the FIX was measured by an APTT (activated partial thromboplastin time) method (Dade Behring, German) using an artificial stimulation of the contact activation as an early stage of intrinsic coagulation pathway, measured with a coagulometer (KC-10A, Heinrich Amelung, German) using a human normal plasma as a standard. The activated FIX (FIXa) was measured by a NAPTT (Non-activated partial thromboplastin time) method (Bio/Data Corp., USA). This is a method measuring coagulation levels caused by the FIXa activity without a contact activation response. The activity of the aFIX was defined as a value subtracting the activity of the FIXa measured by the NAPTT method from the total activity measured by the APTT method. The time for blood clotting differs depending on the diluted concentration of the aFIX, and when the linearity was confirmed in a section of a certain dilution factor, the concentration was calculated after diluting the sample to be in the linear section.

### Example 1: Cloning of recombinant FIX gene and preparation of expression vector

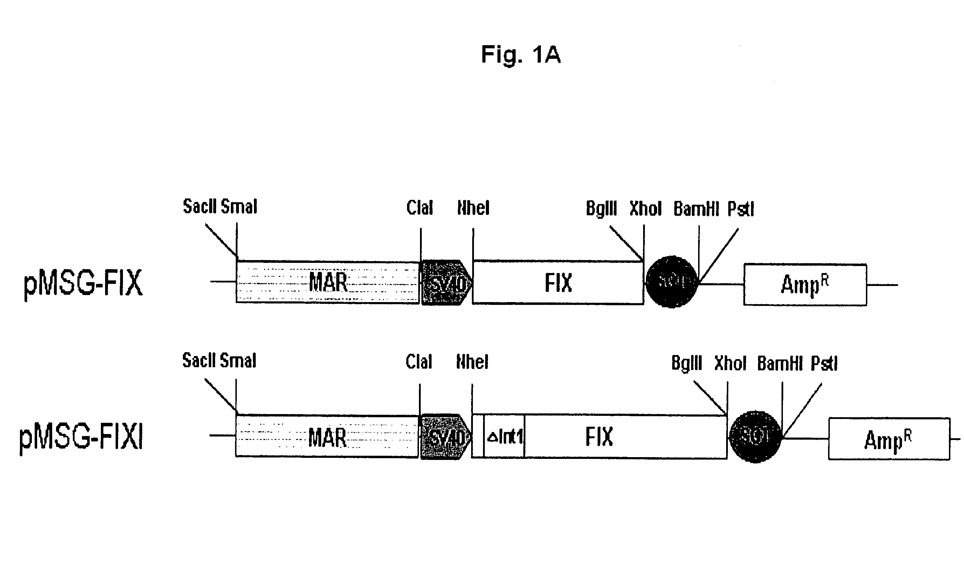

Human liver RNA was extracted, cDNA was synthesized therefrom, and RT-PCR (reverse transcriptase polymerase chain reaction) was performed by using primers of SEQ ID NOs: 3 and 4, which are prepared based on a human FIX gene (GenBank accession No. M11309, 1-2775 bp), to obtain a FIX fragment. Then, the FIX fragment was cloned into *Nhe*I and *Bgl*II restriction enzyme sites of pMSG vector (Accession No.: KCCM 10202; *see* Korea Patent No.10-0408844) developed by the present inventors to obtain pMSG-FIX (Fig. 1A). Further, a 141 bp long segment containing the splicing donor located at 5'-end of the FIX intron 1 and a 157 bp long containing the splicing accepter located at 3'-end were prepared by using genomic PCR. Specifically, a genomic gene was isolated from the human hepatocyte (Clontech, USA). The 5'-end fragment of the human FIX intron 1 was obtained by using primers of SEQ ID NOs: 6 and 7 obtained based on the human FIX genomic gene, and the 3'-end fragment of the human FIX intron 1 was obtained by using primers of SEQ ID NOs: 8 and 9. For the cloning, the primers of SEQ ID NOs: 6 and 7 were designed to have *Sal*I and *M*/*u*I restriction enzymes at their 5'-ends of the fragments, respectively, and the primers of SEQ ID NOs: 8 and 9 were designed to have *M*/*u*I and *Spe*I restriction enzymes at their 5'-ends of the fragments, respectively. Further, in order to insert the human FIX intron 1 fragments into an inherent site thereof, a 5'-side FIX coding gene of the FIX intron 1 was obtained by PCR using primers of SEQ ID NOs: 3 and 10, and 3'-side FIX coding gene of the FIX intron 1 was obtained by PCR using primers of SEQ ID NOs: 11 and 4. In order to insert the FIX intron 1, the primers of SEQ ID NOs: 10 and 11 were prepared to have *Sal*I and *SpeI* restriction enzyme sites, respectively. 4 pieces of genes, which were obtained by PCR and corresponding to the FIX and intron 1, were successively inserted into *Nhe*I and *Bgl*II restriction enzyme sites of pMSG to obtain pMSG-FIXI vector (Fig. 1A).

Full sequence list of the pMSG-FIXI vector was described in SEQ ID NO: 5. The pMSG-FIXI vector has SV40 promoter at 1-419 bp region, the FIX expression gene having a 316 bp long FIX intron 1 fragment in the coding region at 430-2131 bp region, transcription termination region of the gene at 2152-2368 bp region, antibiotics resistance gene (Amp^{R}) at 2974-3834 bp region, and MAR gene at 5067-8035 bp region.

### Example 2: Enhancement effect of FIX expression by intron 1 fragment

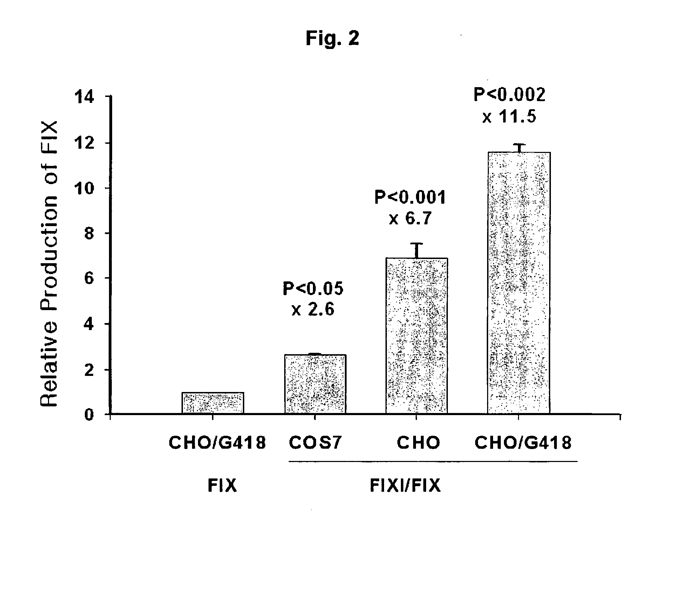

The pMSG-FIX and pMSG-FIXI expression vectors were transfected into COS7 and CHO(DG44) cells by a method using a lipid, respectively. Then, after 48 hours, the amount of the recombinant FIX secreted into a culture supernatant was quantified by ELISA method. The pMSG-FIXI having the obtained FIX intron 1 fragment increased the FIX expression level about 2.6 times in a COS7 cell, and about 6.7 times in a CHO cell, as compared with that of other vector (Fig. 2). Further, 48 hours after co-transfecting the pMSG-FIX and pMSG-FIXI expression vectors with pSVneo vector (Promega, USA), the transfected cells were subcultured in a selection medium (deoxyribonucleic acid and ribonucleic acid-free MEM-a medium, Invitrogen, USA) comprising G418(500 *µ*g/ml), and cultured for about 2 weeks to obtain a stable CHO cell line. In this case, the pMSG-FIXI having the obtained FIX intron 1 fragment increased the FIX expression level about 11.5 times, as compared with that of other vector (Fig. 2).

### Example 3: Enhancement effect of FIX expression by expression vector expressing furin, etc.

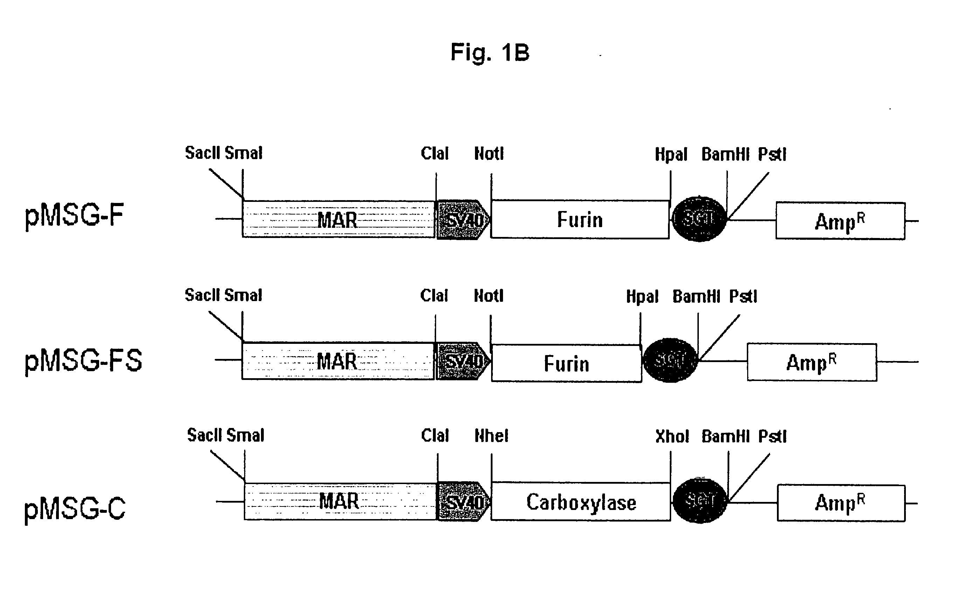

A human furin (pSVLFur, ATCC #79822, GenBank Accession No. X17094) and carboxylase gene (pCMV.hGC+, ATCC # 686) were purchased from ATCC, subcloned into the pMSG vector (Accession No. KCCM 10202; *see* Korea Patent No. 10-0408844) by PCR (polymerase chain reaction) method, and named pMSG-F and pMSG-C, respectively (Fig. 1B). Further, in order to clone a furin protein secreted into extracellular space (i.e., secretor-type furin), PCR was performed to obtain a region excluding a transmembrane region and cytoplasmic tail region of 3'-end of the furin gene, and the region was cloned into the pMSG vector to obtain pMSG-FS expression vector (Fig. 1B).

The pMSG-F, pMSG-FS or pMSG-C expression vector, or combinations of the pMSG-F and pMSG-C or pMSG-FS and pMSG-C were co-transfected with pSVneo vector (Promega, USA), and after 48 hours, the transfected cell was subcloned in the selection medium comprising G418 (500 *µ*g/ml), and cultured for about 2 weeks to obtain a stable CHO cell line. Then, about 24 colonies were selected and cultured to obtain the CHO cell lines expressing furin or carboxylase. The introduction and expression of each gene was confirmed by RT-PCR. In all 5 cases, a cell line having the highest RNA level of furin or carboxylase was selected as a cell line expressing the largest amount of furin or carboxylase, and was used as a CHO cell line for the FIX expression.

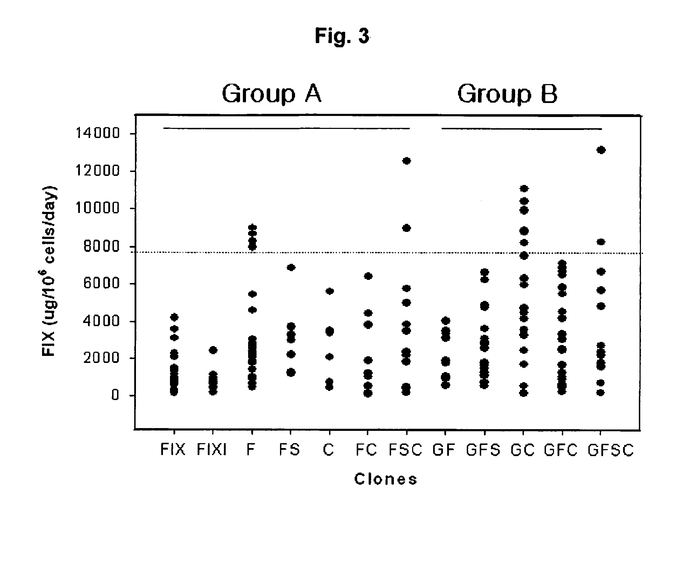

The FIX expression vector pMSG-FIX was transfected into CHO(DG44) cell line as a control, and the pMSG-FIXI was transfected or co-transfected with the furin expression vectors pMSG-F and pMSG-FS and carboxylase expression vector pMSG-C into CHO(DG44) cell line (Group A of Fig. 3), or the pMSG-FIXI was transfected into 5 optimal expression CHO cell lines comprising at least one selected from the group consisting of already obtained furin, secretor-type furin and carboxylase (Group B of Fig. 3). Then, the cells were selected and cultured in a selection medium MEM-α (minimum essential medium-alpha) not comprising a nucleoside and nucleotide to obtain colonies. For gene amplification, pDCH1P plasmid comprising DHFR gene was added thereto together with the cell line during the transfection.

Individual colony was obtained from the formed colonies, and the cell lines expressing the largest amount of FIX were obtained by using an antibody specifically binding to the FIX or ELISA. The selected cell lines overexpressing the FIX were adapted to a condition in which the MTX concentration is successively increased to 0.01, 0.05, 0.25 and 1 µM, for the gene amplification. Further, the formed colonies were collected, the primary gene amplification was performed with MTX, the cell lines were separated individually, the FIX expression level was checked, the overexpressed cell line was obtained, and then further gene amplification was performed. Further, in every gene amplification step, the optional selection process was conducted together to obtain individual cell line, and the selection process to obtain individual cell line was performed again to obtain the final cell line after conducting the gene amplification up to the final MTX concentration of 1 µM.

The recombinant cell lines adapted to 1 µM of MTX and comprising the intron 1 fragment showed generally higher FIX expression pattern than that of the cell lines not comprising the intron 1 fragment (Fig. 3). The cell lines representing the high expression level among the cell lines adapted to 1 µM of MTX showed the FIX productivity of about 8 ~ 14 *µ*g/10⁶ cells/day. 13 representative cell lines representing each groups were selected from the cell lines, and the FIX expression characteristics was analyzed by RT-PCR and western blotting. Then, the selected cell lines were used in the serum-free culture experiment. The FIX productivity of the representative cell lines were summarized in Table 1.

**Table 1 Productivities of cell lines producing FIX**

| Clone | FIX (*µ*g/ml) | Specific productivity of FIX (*µ*g/10⁶ cells/day) |
|---|---|---|
| FIX07 | 2.7 | 2.9 |
| FIXI01(I01) | 2.5 | 3.2 |
| F02 | 4.4 | 6.0 |
| F11 | 6.5 | 7.9 |
| F12 | 6.2 | 8.9 |
| F21 | 4.3 | 9.0 |
| F24 | 4.3 | 8.0 |
| FS09 | 11.9 | 14.3 |
| FS14 | 3.4 | 3.8 |
| GFSC-15 | 2.9 | 4.4 |

### Example 4: Preparation of serum-free medium

In order to prepare 1 L of GC-P1 serum-free medium (Green Cross Corp., Korea), 10.27 g of GC-P1 synthetic medium (serum-free media) was dissolved in 900 mL of deionized distilled water, and 0.75 mL of lipid mixture (Sigma, C-7902, USA) and 2 g of NaHCO₃ (Sigma, S-1554, USA) were successively added thereto and dissolved. Then, pH was titrated to pH 7.2 by using 0.1N HCl or IN NaOH. Deionized distilled water was added to the resulting mixture to adjust the total volume to 1 L. The 1 L medium mixture was filtered by using a 0.22 *µ*m filter (Millipore, USA), and stored at 2~8°C. 10 *µ*g/ml of vitamin K1 (Sigma, USA) sterilized with a filter was added to the medium before the serum-free culture.

### Example 5: Culture of cell lines expressing recombinant FIX in serum-free medium

A suspension culture was performed in an Erlenmeyer Shaker flask or stirring flask by using various commercial serum-free media including the GC-P1 serum-free medium (Green Cross Corp., Korea), HyQ (HyQSFM4; Hyclone, USA), C5467 and C8862 (Sigma), CHO-PMIII and CDmedia (Invitrogen), and protein-free medium (Cellgro). The suspension culture was performed by adding 30 ml of the serum-free medium added with 10 g/mL of vitamin K and 1.0 M of MTX to a 125mL shaker flask, inoculating cells thereto, and stirring the solution at 100 rpm with a stirrer in a 37°C, 5% CO₂ incubator keeping the humidity. 3 ~ 5 subcultures were carried out, and the cell growth was measured to check the adaptation to the suspension culture. The successfully adapted cell lines (I01, F11, F12, F21, F24, FS09, G12, FS14 and GFSC-15) were selected.

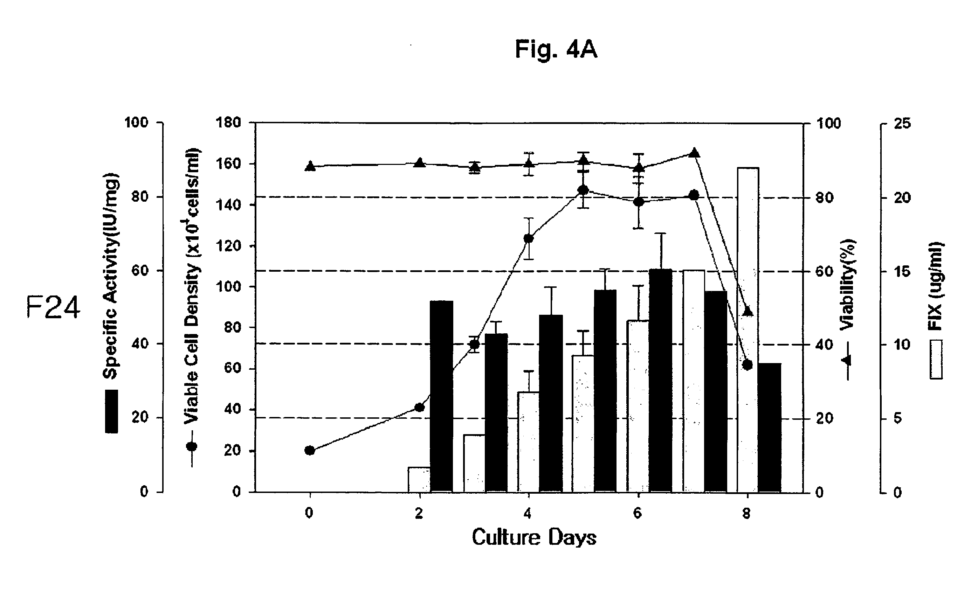

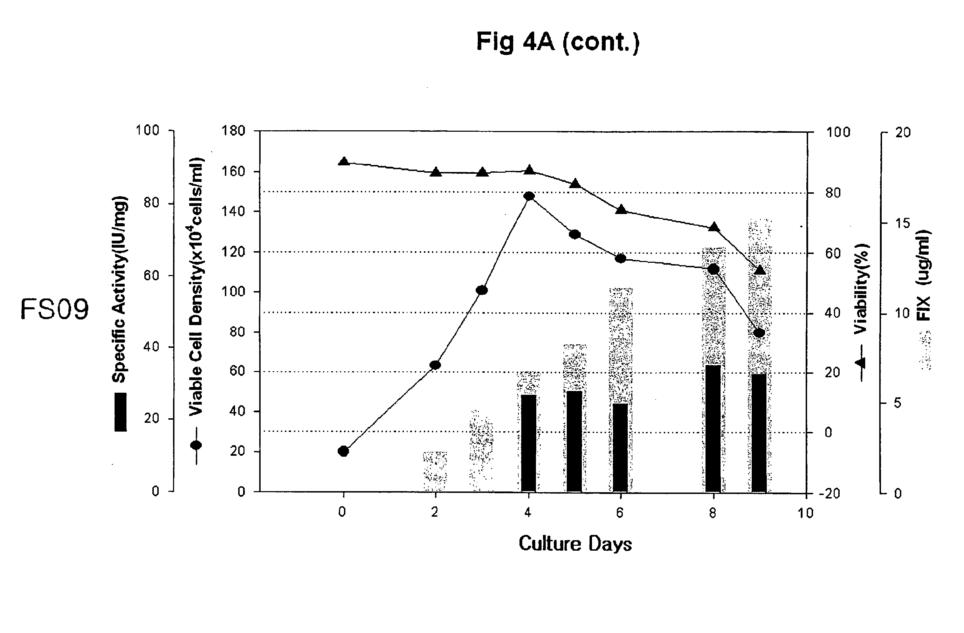

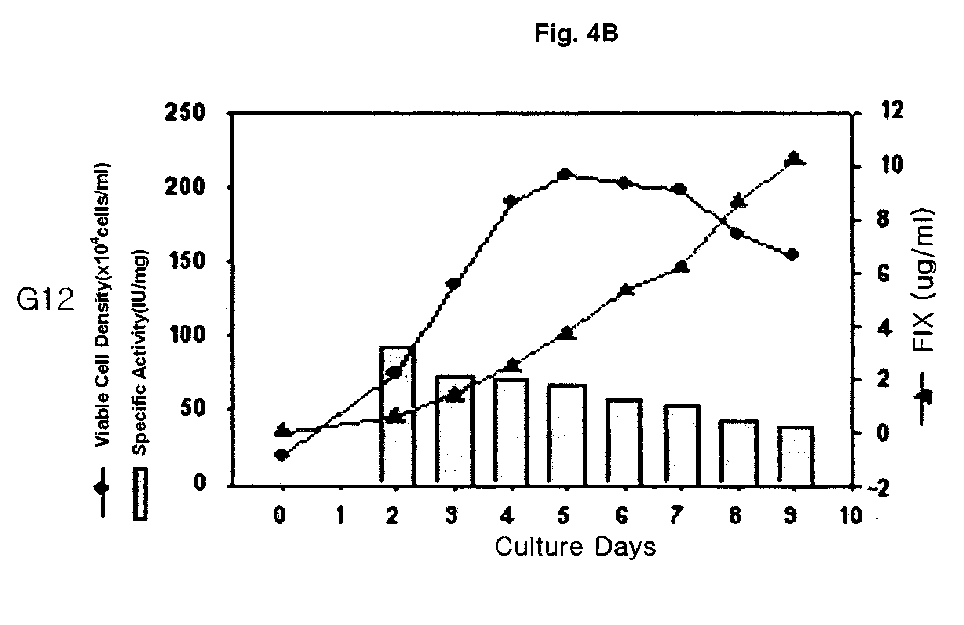

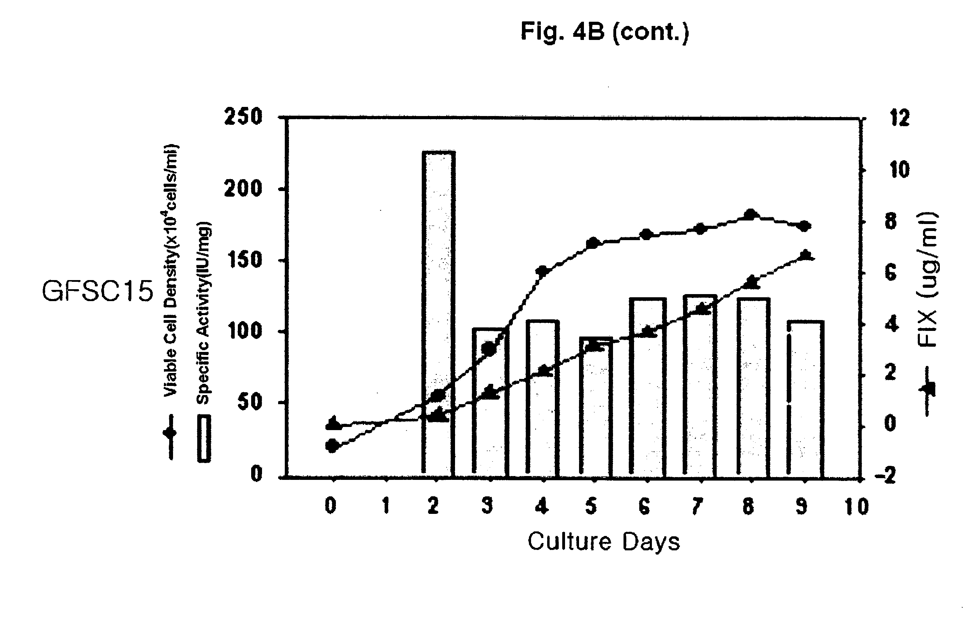

In order to confirm the productivity of the recombinant FIX in the serum-free medium suspension culture of each cell lines, the cell lines were cultured in a shaker or stirring flask for 7~10 days. Then, the amount and activity of the recombinant FIX secreted into a culture supernatant were measured by ELISA and APTT method, respectively. As a result of the 6 day culture, the productivities of FS09, F24, G12 and GFSC-15 cell lines were high, and the specific activities of the recombinant FIX in the cell lines were high as 20 ~ 30 IU/mg in FS09 cell line, 30 ~ 40 IU/mg in F24 cell line (Fig. 4A), 50 - 100 IU/mg in G12 cell line and 100 ~ 150 IU/mg in GFSC-15 cell line (Fig. 4B). It was confirmed that the cell lines producing the recombinant FIX stably grew in the serum-free medium for about 2 -3 months.

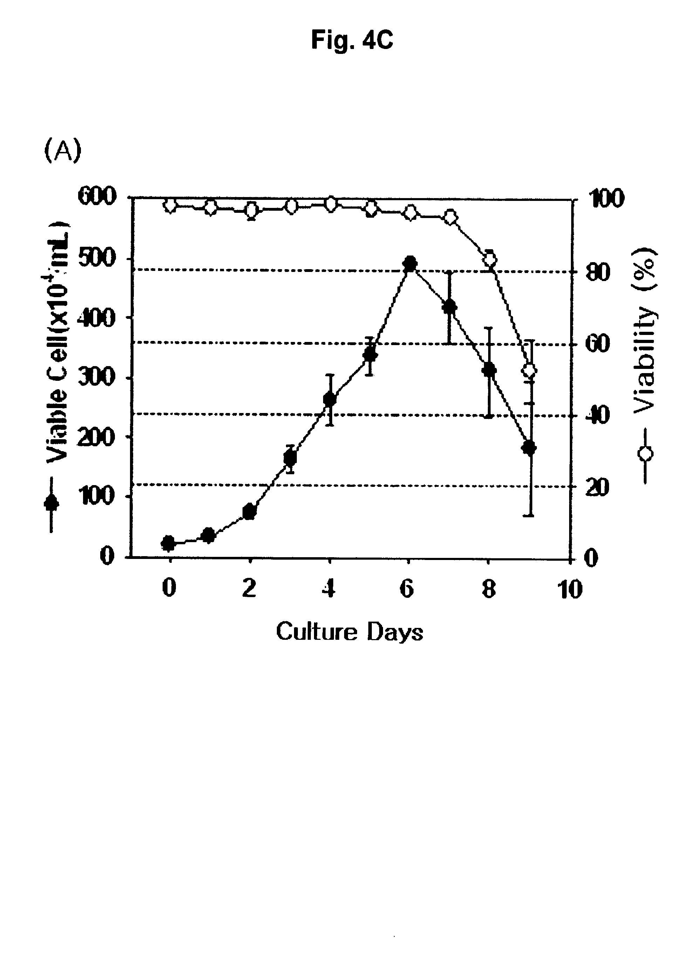

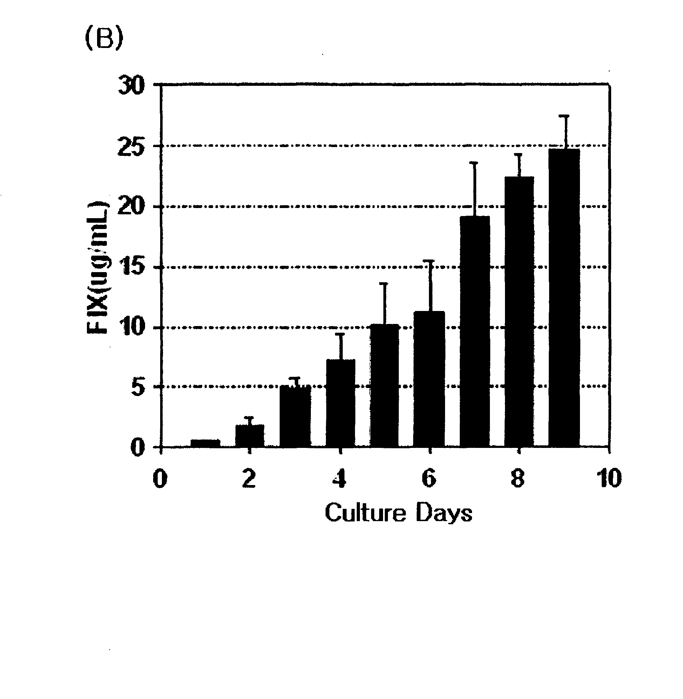

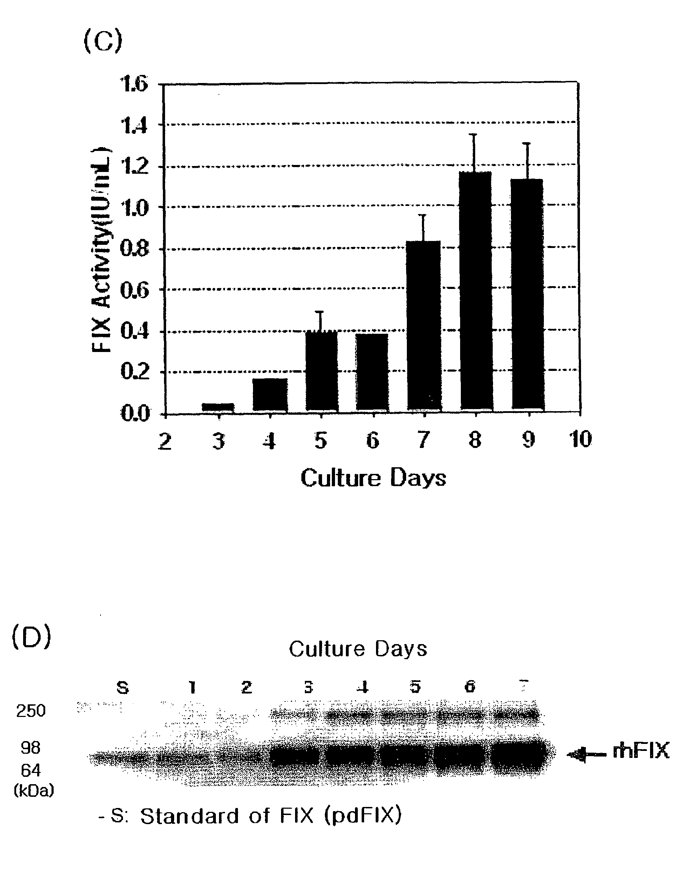

As a result of culturing the F24 cell line using a shaker flask (100 mL) in the GC-P1 serum-free medium, the cell growth was increased up to about 4.0 ~ 5.0 x 10⁶ cells/mL at the 6^{th} day of culture (Fig. 4C (A)), the FIX productivity reached to about 25 *µ*g/ml at the 9^{th} day of the culture (Fig. 4C (B)). The FIX activity was very high as about 1.0~1.2 IU/ml at the 8^{th} ~ 9^{th} day of the culture (Fig. 4C (C)). As a result of western blotting, the productivity of 56 ~ 67 kD FIX was increased according to the culture time (Fig. 4C (D)). This is due to the difference between the glycosylation and carboxylation of the FIX. When the F24 cell line was cultured in a stirring flask with the GC-P1 serum-free medium, as a result of analyzing the samples of the 4^{th}, 6^{th}, and 8^{th} day of culture, the activity level of the aFIX was increased according to the culture time, and the ratio of the FIXa to the total was about 1 ~ 8%.

### Example 6: Enhancement effect of FIX expression by adding hydrolyzed product in serum-free medium

The cell growth of the F24 cell line, the productivity and activity of the FIX were examined after adding various hydrolysates to the GC-P1 serum-free medium to increase the FIX productivity in the medium. Each 5 g/L of soy hydrolysate (Quest International, USA), yeast hydrolysate (Yeastolate, BD Science, USA) and wheat hydrolysate (Quest International, USA) were added to the GC-P1 serum-free medium, and the cell growth of F24 cell line and FIX productivity were compared with those of the group using GC-P1 medium only.

For serum-free culture, each 30ml of the GC-P1 serum-free mediums prepared by the method of Example 4 were put into 125mL shaker flasks, and the F24 cell lines which were transfected to express the FIX, were inoculated thereto at the early concentration of 2 x 10⁵ cells/ml. Then, the culture was carried out at 120 rpm in a 37°C, 5% CO₂ incubator. 1 ml of the culture solution was collected from each flask everyday after starting the culture, diluted in a tryphan blue solution, and the cell number and viability were measured by using a hematocytometer and microscope. Further, the amount of FIX in the culture solution was measured with a human FIX ELISA kit.

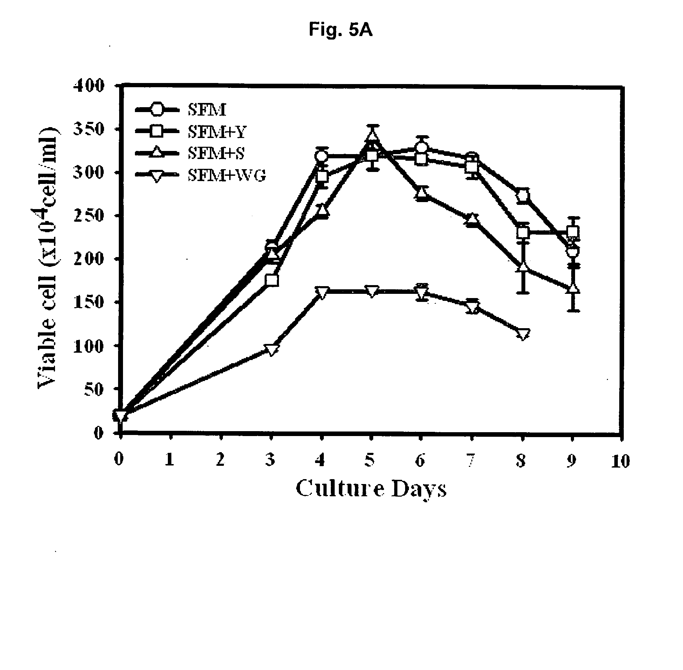

Fig. 5A is a result showing the cell growths after adding hydrolysates to the GC-P1 medium according to the culture time. The cell growth in the medium added with a wheat hydrolysate was stopped at 1.7 x 10⁶ cells/ml at the 4^{th} day of culture, while the cell growth in the medium added with a yeast hydrolysate was similar to that of the culture using the GC-P1 basic medium only. Further, the cell growth in the medium added with a soy hydrolysate was similar to that of the culture using the GC-P1 basic medium at the early stage, but it was rapidly decreased at the late stage. Finally, the cell growth in the medium added with the yeast hydrolysate was maintained up to 3*~*3.5 x 10⁶cells/ml.

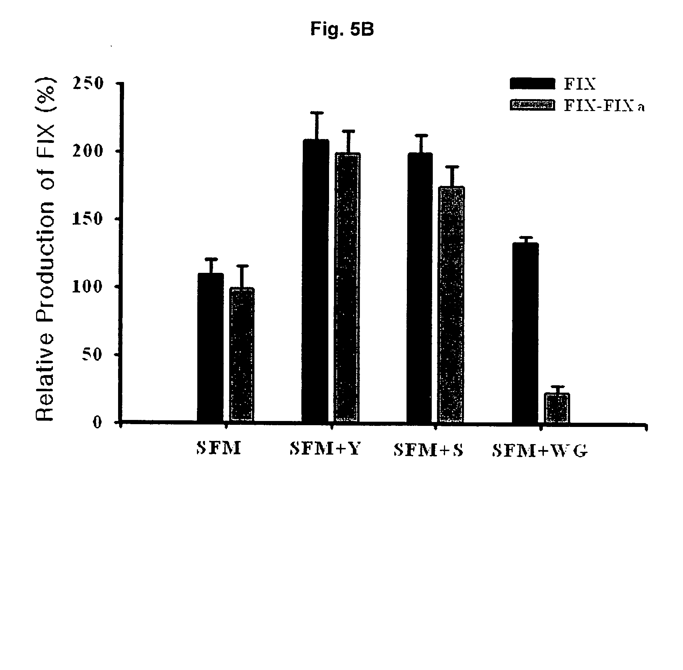

Fig. 5B is a result showing the FIX productivity in the serum-free culture after adding hydrolysate to GC-P1 medium at the 8^{th} day of the culture comparing with that of the culture using the GC-P1 basic medium only. The FIX productivity in the culture added with the yeast hydrolysate having the high cell growth was increased about 1.8~2.0 times, as compared with that of the culture using the GC-P1 basic medium only. The FIX productivity in the soy hydrolysate-added culture was about 10% lower than that of the yeast hydrolysate-added culture not making big difference. However, the FIX productivity in the wheat hydrolysate-added culture was low, and most of the FIX product could not be used due to their modification to the FIXa. Consequently, the aFIX production could be enhanced only in the yeast or soy hydrolysate-added culture.

### Example 7: Serum-free culture by adding mixture of yeast and soy hydrolysates according to the mixing ratio

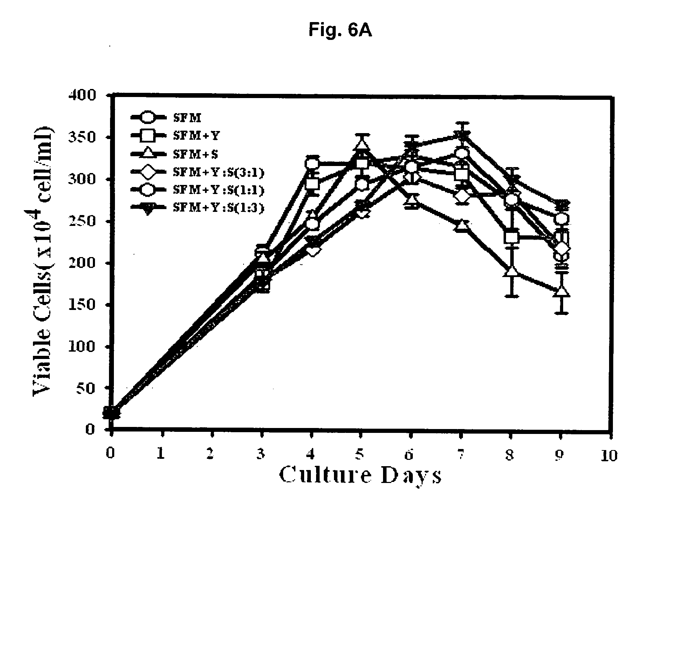

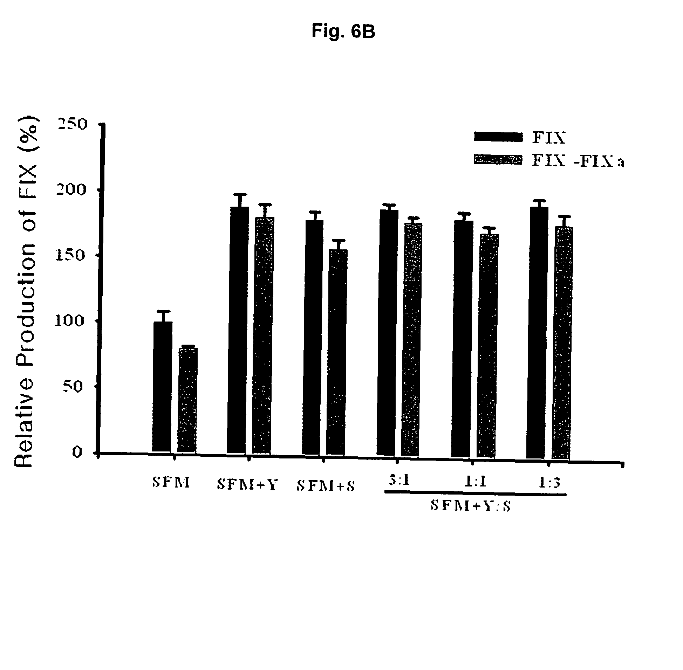

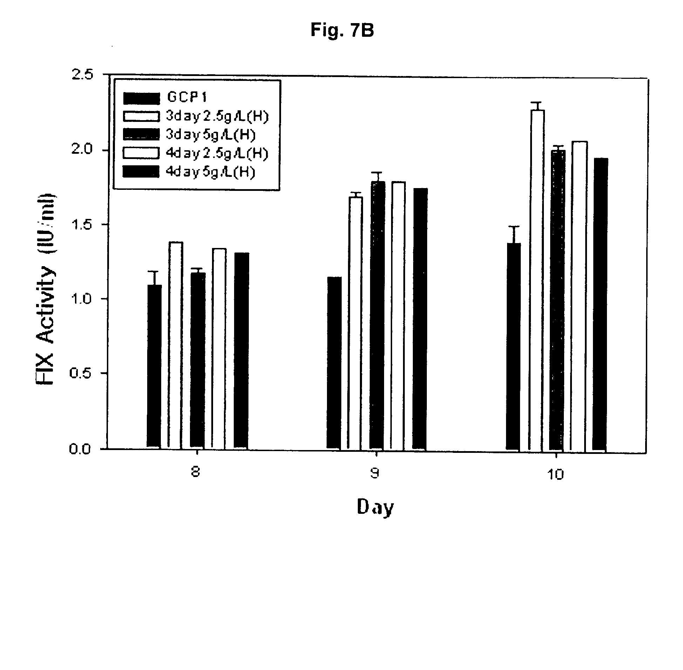

The optimal mixing ratio of the yeast hydrolysate (Y) and soy hydrolysate (S), which are effective in the serum-free culture of the F24 cell line, was searched to optimize the production process. The 3:1, 1:1 and 1:3 mixture of the yeast hydrolysate (Y) and soy hydrolysate (S) were added to the GC-P1 medium, respectively, and the growth of the cultured cell (Fig. 6A) and FIX productivity (Fig. 7B) were examined. As a result, when the soy hydrolysate (S) was used only, the cell number was little low due to the fast cell death at the late culture, but the cell growths had no difference regardless of the mixing ratio of the two hydrolysates in another conditions. This was also found in the secretory production of the FIX (Fig. 6B).

Consequently, when the yeast hydrolysate (Y) and soy hydrolysate (S) were used independently or in a proper combination in the GC-P1 basic medium, specifically, when the yeast hydrolysate was added 25%~100% (w/w) based on the amount of soy hydrolysate in the mixture, the FIX productivity was increased about 1.8~2.2 times.

### Example 8: Serum-free culture according to the amount of added yeast hydrolysate

In order to optimize the culture condition of the serum-free culture of F24 cell line in the GC-P1 medium added with the yeast hydrolysate, the changes of the cell growth and FIX productivity according to the addition time and amount of the yeast hydrolysate were examined. The culture in the yeast hydrolysate-added medium was compared with the culture in the medium not comprising the hydrolysate in which the culture was performed under 4 kinds of conditions, i.e., the addition point was the 3^{rd} and 4^{th} day of the culture (exponential phase), and the concentration was 2.5 g/L and 5.0 g/L.

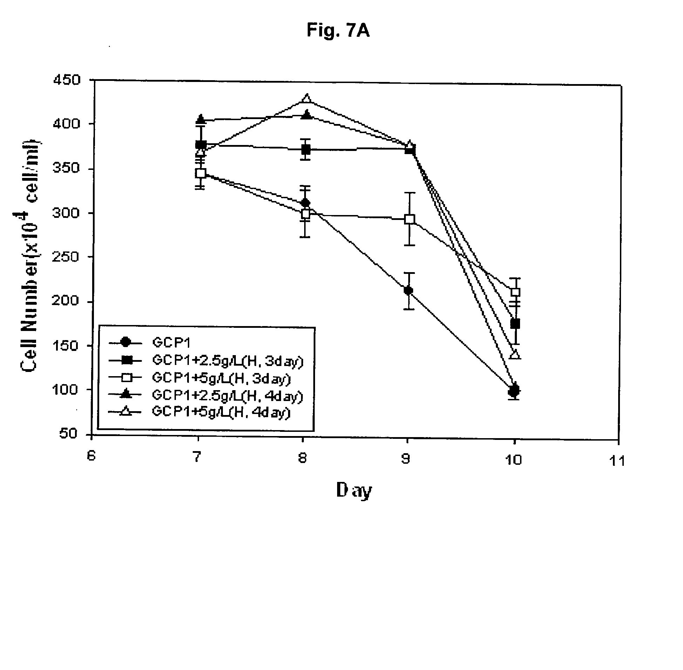

As a result, when 5.0 g/L of yeast hydrolysate was added at the 3^{rd} day of the culture, the cell growth was somewhat dully increased, as compared with that under other conditions (Fig. 7A), and the FIX activities did not differ depending on the addition amount and time, and generally increased about 70-80% and more than the culture in the GC-P1 basic medium (Fig. 7B).

### Example 9: Productivity of FIX in serum-free culture added with yeast hydrolysate according to osmotic pressure of culture medium

In order to inhibit the production of the FIXa, which was partially produced with the increase of time, in the serum-free culture using the GC-P1 basic medium added with 5.0 g/L of yeast hydrolysate, the change caused by the increase of osmotic pressure, which is induced by adding NaCl during cell culture, was examined.

As a result, according to the increase of osmotic pressure, the total productivity of the FIX was not changed because the cell growth was decreased while the specific productivity of the FIX per cell was increased. Particularly, the FIXa production was inhibited without reducing the FIX productivity when osmotic pressure was increased up to 350-388 mOsm at the late culture. However, the excessively high osmotic pressure was not suitable because it reduced the total productivity by excessively inhibiting the cell growth. Table 2 shows the relative FIX productivity in other conditions when the FIX productivity in the GC-P1 serum-free medium having about 312 mOsm of the osmotic pressure was defined as 100%.

**Table 2: Comparison of FIX production characteristics according to osmotic pressure of serum-free medium**

| | Osmotic pressure of medium (mOsm) | | | | |
|---|---|---|---|---|---|
| | 312 | 349 | 388 | 459 | 533 |
| FIX activity (%) | 100.0 | 102.1 | 99.1 | 63.8 | 38.5 |
| Activated FIX (FIXa) (%) | 48.4 | 22.9 | 8.3 | 3.0 | 3.7 |

### Example 10: Productivity of FIX according to the Ca²⁺ concentration in GC-P1 serum-free medium added with yeast hydrolysate

The enzymatic activity of FIX needs a calcium ion (Ca²⁺). As described in the present invention, the influence of additional addition of calcium ion (Ca²⁺) in the form of calcium chloride (CaCl₂) to the GC-P1 basic medium added with 5.0 g/L of the yeast hydrolysate on the cell growth of F24 cell line and FIX productivity during the serum-free culture was examined (Table 3).

In order to examine the productivity of the FIX which was secreted into a culture supernatant, in F24 cell line culture, the FIX activity and activation level in the culture supernatant of the 7^{th} day of the culture were measured by APTT and NAPTT methods, and compared (Table 3). The FIX production in the Ca²⁺-free medium was similar to that in the medium having Ca²⁺, but the produced FIX did not have the activity. When the concentration of Ca²⁺ was increased from 0 mM to 2.6 mM by adding calcium chloride, the aFIX was produced and the amount thereof was gradually increased. Table 3 shows the FIX production characteristics according to the calcium chloride concentration based on the FIX productivity when the calcium concentration of the GC-P1 medium was controlled to 1.0 mM (herein after 'control'). The aFIX productivity was increased about 30% at the Ca²⁺ concentration of 1.0~1.3 mM in case that the FIXa was kept not to be increased. Further, at the Ca²⁺ concentration of 2.6 mM, generally, the aFIX productivity was reached to the maximum level in which the aFIX productivity was about 1.6 times higher than that in the GC-P1 medium (aFIX = FIX-FIXa. In Table 3, because the FIX productivity at the Ca²⁺ concentration of 2.6 mM was increased about 100%, and the FIXa productivity was increased about 40%, as compared with that of control, the aFIX productivity was substantially increased about 60%, i.e., about 1.6 times, as compared with that of control.), but already activated FIX was also produced a lot.

**Table 3 Comparison of FIX production characteristics according to the Ca²⁺ concentration of serum-free medium**

| | CaCl₂ (mM) | | | | |
|---|---|---|---|---|---|
| | 1.0 | 1.15 | 1.3 | 1.8 | 2.6 |
| Production of FIX (%) | 100 | 118 | 132 | 167 | 200 |
| Production of activated FIX (FIXa) (%) | 0.1 | 0.2 | 0.2 | 13.8 | 41.7 |

### Example 11: Optimized culture condition for maximum production of aFIX

For maximum production of the aFIX, a process producing an aFIX was obtained by applying the effective culture conditions described in the above Examples in combination. First, cells were inoculated to the GC-P1 serum-free medium added with 2.5 ~ 5 g/L of the yeast hydrolysate and 1.0 ~ 1.3 mM of calcium chloride (CaCl₂) as a source of calcium ion with the optimal early inoculation concentration of 5x10⁵ cells/ml (general inoculation concentration: 2x10⁵ cells/ml), and cultured under a proper high osmotic pressure condition (350~388 mOsm). As a result, the aFIX productivity was increased about 2.2~3.5 times, as compared with that in GC-P1 basic medium (Table 4).

**Table 4 Analysis of optimized production process characteristics of FIX by combinational control of medium additive and culture condition in a serum-free medium**

| Medium condition | SFM +general injection conc. | SFM +yeast hydrolysate +general injection conc. | SFM + yeast hydrolysate +optimal injection conc. | SFM + yeast hydrolysate + optimal injection conc. +Ca²⁺ |
|---|---|---|---|---|
| Relative production of rhFIX (%) | 100 | 188.5 | 229.6 | 310.0 |
| Activated (FIXa) (%) | 19.5 | 10.9 | 21.5 | 30.5 |
| Multiple in the increase ^{a} of relative active FIX (aFIX) | 1.0 | 2.2 | 2.6 | 3.5 |

| | | | | |
|---|---|---|---|---|
| a: Multiple in the increase of relative active FIX (aFIX) = aFIX production of test group (FIX-FIXa) / aFIX production of control group (FIX-FIXa) | | | | |

### Industrial Applicability

As suggested in the present invention, the productivity of a recombinant protein comprising the FIX may be increased by using the proper combination of hydrolysates. Further, the productivity of aFIX may be maximized through using a suitable hydrolysate, treating a proper osmotic pressure, optimizing the Ca²⁺ concentration and developing a process optimizing the conditions.

This process may be used to increase the productivity of the recombinant protein having a FIX-like feature and the secretion of the active factor. Particularly, the process may be applied for increasing the productivity of the active protein when the process is used to culture a cell line expressing vitamin K-dependent recombinant proteins (blood coagulation factors V, VII and IX, proteins C and S, and the like).
## Claims

1. A method for manufacturing human blood coagulation factor IX (FIX) by culturing an animal cell transfected with a recombinant expression vector expressing human FIX in a serum-free medium for animal cell culture, wherein the serum-free medium contains a yeast hydrolysate at a concentration of 2.5 to 5 g/L, and a calcium ion at a concentration of 1.0 to 1.3 mM, and has an osmotic pressure ranging from 350 to 388 mOsm.

2. The method of claim 1, wherein the recombinant expression vector comprises:
a nucleotide sequence of human β-globin MAR element having the nucleotide sequence of SEQ ID NO: 1; and
a nucleotide sequence of human FIX having a fragment of intron 1 in place of the inherent intron 1 of the nucleotide sequence encoding human FIX, wherein the fragment of intron 1 is composed of a 141 bp to 981 bp long nucleotide sequence having the 5'-end splicing donor of intron 1 fused with a 157 bp to 444 bp long nucleotide sequence having the 3'-end splicing acceptor of intron 1; and the fragment of intron 1 is a 316 bp long nucleotide sequence.

3. The method of claim 2, wherein the fragment of intron 1 has the nucleotide sequence of SEQ ID NO: 2.

4. The method of claim 2, wherein the recombinant expression vector has the nucleotide sequence of SEQ ID NO: 5.

5. The method of claim 1, wherein the animal cell is CHO, BHK or COS7.

6. The method of claim 1, wherein the animal cell is further transfected with at least one vector selected from the group consisting of expression vectors expressing furin, secretor type furin and carboxylase, respectively.

7. A serum-free medium for manufacturing human blood coagulation factor IX (FIX), which is a serum-free medium for animal cell culture having a yeast hydrolysate at a concentration of 2.5 to 5 g/L, an osmotic pressure ranging from 350 to 388 mOsm, and a calcium ion at a concentration of 1.0 to 1.3 mM.

## Patentansprüche

1. Verfahren zum Herstellen von menschlichem Blutgerinnungsfaktor IX (FIX) durch Züchten einer tierischen Zelle, die mit einem rekombinanten, menschlichen FIX exprimierenden Expressionsvektor transfiziert ist, in einem serumfreien Medium für tierische Zellkultur, wobei das serumfreie Medium ein Hefe-Hydrolysat in einer Konzentration von 2,5 bis 5 g/l und ein Calcium-Ion in einer Konzentration von 1,0 bis 1,3 mM enthält und einen osmotischen Druck aufweist, der von 350 bis 388 mOsm reicht.

2. Verfahren nach Anspruch 1, wobei der rekombinante Expressionsvektor umfasst:
eine Nucleotidsequenz des menschlichen β-Globin MAR-Elements mit der Nucleotidsequenz der SEQ ID NO: 1; und
eine Nucleotidsequenz des menschlichen FIX mit einem Fragment von Intron 1 anstelle des eigenen Introns 1 der Nucleotidsequenz, die menschlichen FIX kodiert, wobei das Fragment von intron 1 aus einer 141 bp bis 981 bp langen Nucleotidsequenz mit dem Spleißdonor des 5'-Endes von Intron 1, fusioniert mit einer 157 bp bis 444 bp langen Nucleotidsequenz mit dem Spleißakzeptor des 3'-Endes von Intron 1 zusammengesetzt ist; und es sich bei dem Fragment von Intron 1 um eine 316 bp lange Nucleotidsequenz handelt.

3. Verfahren nach Anspruch 2, wobei das Fragment von Intron 1 die Nucleotidsequenz der SEQ ID NO: 2 aufweist.

4. Verfahren nach Anspruch 2, wobei der rekombinante Expressionsvektor die Nucleotidsequenz der SEQ ID NO: 5 aufweist.

5. Verfahren nach Anspruch 1, wobei es sich bei der tierischen Zelle um CHO, BHK oder COS7 handelt.

6. Verfahren nach Anspruch 1, wobei die tierische Zelle ferner mit mindestens einem Vektor transfiziert ist, ausgewählt aus der Gruppe, bestehend aus Expressionsvektoren, die Furin, Furin des sekretorischen Typs bzw. Carboxylase exprimieren.

7. Serumfreies Medium zum Herstellen von menschlichem Blutgerinnungsfaktor IX (FIX), wobei es sich um ein serumfreies Medium für tierische Zellkultur mit einem Hefe-Hydrolysat in einer Konzentration von 2,5 bis 5 g/l, einem osmotischen Druck, der von 350 bis 388 mOsm reicht, und einem Calcium-Ion in einer Konzentration von 1,0 bis 1,3 mM handelt.

## Revendications

1. Procédé de production du facteur IX de la coagulation sanguine humain (FIX) par culture d'une cellule animale transfectée avec un vecteur d'expression recombinant exprimant le FIX humain dans un milieu sans sérum destiné à la culture de cellules animales, le milieu sans sérum contenant un hydrolysat de levure à une concentration de 2,5 à 5 g/L et un ion calcium à une concentration de 1,0 à 1,3 mM, et ayant une pression osmotique allant de 350 à 388 mOsm.

2. Procédé selon la revendication 1, le vecteur d'expression recombinant comprenant :
une séquence nucléotidique d'élément MAR de β-globine humaine comprenant la séquence de nucléotides SEQ ID NO : 1 ; et
une séquence nucléotidique du FIX humain comprenant un fragment d'intron 1 à la place de l'intron 1 inhérent à la séquence nucléotidique codant pour le FIX humain, le fragment d'intron 1 étant composé d'une séquence nucléotidique de longueur 141 bp à 981 bp, comprenant le donneur d'épissage de l'extrémité 5' de l'intron 1, fusionnée avec une séquence nucléotidique de longueur 157 bp à 444 bp comprenant l'accepteur de l'extrémité 3' de l'intron 1 ; et le fragment de l'intron 1 étant une séquence nucléotidique de longueur 316 bp.

3. Procédé selon la revendication 2, le fragment de l'intron 1 ayant la séquence nucléotidique SEQ ID NO : 2.

4. Procédé selon la revendication 2, le vecteur d'expression recombinant ayant la séquence nucléotidique SEQ ID NO : 5.

5. Procédé selon la revendication 1, la cellule animale étant CHO, BHK ou COS7.

6. Procédé selon la revendication 1, la cellule animale étant en outre trans-féctée avec au moins un vecteur choisi dans le groupe constitué de vecteurs d'expression exprimant respectivement la furine, un sécréteur de type furine et une carboxylase.

7. Milieu sans sérum destiné à la production du facteur IX de la coagulation sanguine humain (FIX), qui constitue un milieu sans sérum pour la culture de cellules animales comprenant un hydrolysat de levure à une concentration de 2,5 à 5 g/L, une pression osmotique allant de 350 à 388 mOsm et un ion calcium à une concentration de 1,0 à 1,3 mM.
